Europäisches Patentamt

European Patent Office

Office européen des brevets

(19)

(11) **EP 1 275 642 A1**

(12) **EUROPEAN PATENT APPLICATION**
published in accordance with Art. 158(3) EPC

(43) Date of publication:
**15.01.2003 Bulletin 2003/03**

(51) Int Cl.⁷: **C07C 327/32**

(21) Application number: **01921936.9**

(86) International application number:
**PCT/JP01/03383**

(22) Date of filing: **20.04.2001**

(87) International publication number:
**WO 01/081300 (01.11.2001 Gazette 2001/44)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **21.04.2000 JP 2000120515**

(71) Applicant: **KANEKA CORPORATION
Osaka-shi, Osaka 530-8288 (JP)**

(72) Inventors:
• **KINOSHITA, Koichi
Kakogawa-shi, Hyogo 675-0057 (JP)**
• **YAMASHITA, Koki
Kobe-shi, Hyogo 655-0872 (JP)**
• **UEDA, Yasuyoshi
Himeji-shi, Hyogo 671-1227 (JP)**

(74) Representative: **Hubert, Philippe et al
Cabinet Beau de Loménie
158, avenue de l'Université
75340 Paris Cédex 07 (FR)**

(54) **PROCESS FOR CRYSTALLIZATION OF 2-ACETYLTHIO-3-PHENYL-PROPIONIC ACID**

(57)    The present invention provides an industrial method for recovering and purifying 2-acetylthio-3-phenylpropionic acid, particularly an optically active 2-acetylthio-3-phenylpropionic acid, as crystals of the free acid.

2-Acetylthio-3-phenylpropionic acid is recovered and purified as crystals of the free acid by using an aliphatic hydrocarbon solvent and/or an aromatic hydrocarbon solvent.

EP 1 275 642 A1

**Description**

TECHNICAL FIELD

The present invention relates to a crystallization method of 2-acetylthio-3-phenylpropionic acid which is of use as an intermediate for production of pharmaceuticals and the like. The optically active 2-acetylthio-3-phenylpropionic acid, particularly (S)-2-acetylthio-3-phenylpropionic acid, is of use as an intermediate for production of antihypertensive drugs (Japanese Kokai Publication Hei-8-337527).

BACKGROUND ART

As the method for obtaining 2-acetylthio-3-phenyl-propionic acid, there is known the method which comprises reacting (R)-2-bromo-3-phenylpropionic acid with potassium thioacetate to give (S)-2-acetylthio-3-phenylpropionic acid and recovering and purifying the same as crystals of the corresponding dicyclohexylamine salt (JP-A-08-337527, JP-A-07-48259, JP-A-07-300479, JP-A-07-196658, JP-A-06-56790).

However, in this method, when (S)-2-acetylthio-3-phenylpropionic acid is submitted to a subsequent reaction, said dicyclohexylamine salt need to be converted to a free acid (JP-A-08-337527, JP-A-07-48259, JP-A-07-300479, JP-A-07-196658, JP-A-06-56790) and problems occur such as procedural complexity, the need for treatment of the nitrogen-containing compound, and additional cost and so forth. It goes without saying, therefore, that if (S)-2-acetylthio-3-phenylpropionic acid could be recovered and purified as the free acid, it is quite a benefit.

The known technology of recovering (S)-2-acetylthio-3-phenylpropionic acid as a free acid includes:

(1) the method which comprises converting said dicyclohexylamine salt to a free acid and concentrating the ethyl acetate solution containing the free acid to give the free acid as an oil (JP-A-07-196658, JP-A-06-56790) and

(2) the method which comprises converting said dicyclohexylamine salt to a free acid, concentrating the ethyl acetate solution containing the free acid, stripping the concentrate with dichloromethane (twice), and drying it under reduced pressure overnight to give the free acid as a crystalline solid (JP-A-08-337527).

However, both methods involve conversion of the dicyclohexylamine salt once recovered to the free acid, namely, the free acid is not obtained directly, and processes of concentration through drying under reduced pressure for obtaining a crystalline solid is very difficult to carry out on an industrial scale.

In addition, a preliminary investigation by the present inventors revealed that in the endeavoring to recover (S)-2-acetylthio-3-phenylpropionic acid as crystals of the free acid, a crystalline solid may at any rate be obtained by allowing the oil obtained in the above method (1) to stand for a long time or removing the coexisting solvent almost thoroughly by using the above method (2), but it was found that if one attempts to crystallize (S)-2-acetylthio-3-phenylpropionic acid in solution (crystallization), the solution tends to become oily or the oil so formed solidifies to prevent withdrawal of the product from the crystallizer, so that it is extremely difficult to crystallize it in a good condition.

Thus, it has a very important meaning to establish an industrial method for recovering and purifying 2-acetylthio-3-phenylpropionic acid, particularly an optically active 2-acetylthio-3-phenylpropionic acid, as crystals of the free acid.

SUMMARY OF THE INVENTION

The present invention, in view of the above-mentioned state of the art, has for its object to provide an industrial method for recovering and purifying 2-acetylthio-3-phenylpropionic acid, particularly an optically active 2-acetylthio-3-phenylpropionic acid, as crystals of the free acid.

The inventors of the present invention explored in earnest to solve the above problem and found that 2-acetylthio-3-phenylpropionic acid can be recovered and purified as crystals in good condition by using a solvent substantially composed of an aliphatic hydrocarbon solvent and/or an aromatic hydrocarbon solvent.

The present invention, therefore, is a crystallization method of 2-acetylthio-3-phenylpropionic acid which comprises crystallizing 2-acetylthio-3-phenylpropionic acid by using a solvent substantially composed of an aliphatic hydrocarbon solvent and/or an aromatic hydrocarbon solvent as a crystallization solvent.

The present invention is now described in detail.

DETAILED DESCRIPTION OF THE INVENTION

2-Acetylthio-3-phenylpropionic acid for use in the present invention can be synthesized by, for example, reacting 2-halo-3-phenylpropionic acid with a salt of thioacetic acid. 2-Acetylthio-3-phenylpropionic acid may be an optically active compound, and an optically active 2-acetylthio-3-phenylpropionic acid, for example (S)-2-acetylthio-3-phenylpropionic acid, can be synthesizedby reacting (R)-2-halo-3-phenylpropionic acid with a salt of thioacetic acid, while (R)-2-acetylthio-3-phenylpropionic acid can be synthesized by reacting (S)-2-halo-3-phenylpropionic acid with a salt of thioacetic acid.

The 2-halo-3-phenylpropionic acid mentioned above is not particularly restricted but 2-chloro-3-phenylpropionic acid or 2-bromo-3-phenylpropionic acid is preferably used.

The salt of thioacetic acid mentioned above is not particularly restricted but is preferably an alkali metal thioacetate, and there can be mentioned, for example, sodium thioacetate, potassium thioacetate, lithium thioacetate, and cesium thioacetate, etc. Particularly preferred among them is potassium thioacetate. The above salt of thioacetic acid may be prepared from thioacetic acid and a base (e.g. a hydroxide, hydride or alkoxide of an alkali metal) in the reaction system and used.

Specifically, for example, the above 2-acetylthio-3-phenylpropionic acid (e.g. (S)-2-acetylthio-3-phenylpropionic acid) can be synthesized by reacting the above 2-halo-3-phenylpropionic acid (e.g. (R)-2-chloro-3-phenylpropionic acid) with at least one equivalent (preferably 1 to 2 equivalents) of the above salt of thioacetic acid (e.g. potassium thioacetate) in a suitable solvent (e.g. dimethylformamide) at a suitable temperature condition (e.g. 0 to 50°C).

In order to inhibit formation of oxidation byproducts, such as a disulfide form which is the oxidation product of the reactionbyproduct, namely, 2-mercapto-3-phenylpropionic acid, the above reaction is preferably conducted under an inert gas atmosphere.

By the above reaction, several kinds of impurities may be produced as byproducts, in some instances, but in consideration of the ease of removal of the impurity in the extraction and crystallization procedure and in consideration of the target purity of 2-acetylthio-3-phenylpropionic acid crystals, the reaction solution may be incubated for ripening so as to reduce impurities which are not easy to remove among the above-mentioned impurities. The ripening mentioned above means a procedure of maintaining the reaction solution in stationary condition or under stirring at a certain temperature for a certain time period following a substantial or thorough completion of the reaction. The incubation temperature for ripening is not particularly restricted but may be 0 to 50°C, for example. The incubation time for ripening is usually within 48 hours. Needless to say, the incubation temperature and time for ripening can be varied according to the purpose.

The 2-acetylthio-3-phenylpropionic acid in the reaction solution is extracted with an organic solvent under acidic conditions (e.g. pH 0 to 6, preferably pH 1 to 4) and subjected to crystallization.

Preferably, for example, the solution obtainable by extracting the reaction solution with an aromatic hydrocarbon solvent, or a concentrate thereof, or the solution obtainable by extracting the reaction solution with an organic solvent other than an aromatic hydrocarbon solvent and subjecting the resulting solution to solvent exchange to ultimately give a solution of an aromatic hydrocarbon solvent, or a concentrate thereof, is subjected to crystallization. Needless to say, the aromatic hydrocarbon solvent to be used in this procedure is preferably the same aromatic hydrocarbon solvent as that to be used for crystallization.

The above-mentioned solution or concentrate may be the solution stripped of contaminants by washing with water or with each of various aqueous solutions in the extraction procedure as an ordinary procedure or, where necessary, may be the solution obtained by causing 2-acetylthio-3-phenylpropionic acid to be distributed into a water phase to remove the contaminants into an organic solvent under neutral to basic conditions (e.g. pH 7 to 11, preferably pH 8 to 10) and finally causing 2-acetylthio-3-phenylpropionic acid to be distributed into an organic solvent under acidic conditions (e.g. pH 0 to 6, preferably pH 1 to 4) to remove the contaminants inclusive of salts formed by neutralization into a water phase.

Furthermore, as a preferred procedure, 2-acetylthio-3-phenylpropionic acid, a solution containing 2-acetylthio-3-phenylpropionic acid, or a concentrate thereof may be treated with an adsorbent such as activated carbon or the like for removal of contaminants and for decolorization or deodorization prior to crystallization, if necessary.

The 2-acetylthio-3-phenylpropionic acid thus obtained can be crystallized by using an aliphatic hydrocarbon solvent and/or an aromatic hydrocarbon solvent.

The crystallization method of the invention can be used not only for recovering 2-acetylthio-3-phenylpropionic acid from the reaction solution but also for recrystallization purposes. Moreover, it can also be used for the purpose of isolating 2-acetylthio-3-phenylpropionic acid as crystals of the free acid from the salt of 2-acetylthio-3-phenylpropionic acid with a base (e.g. above-mentioned dicyclohexylamine salt) .

The solvent for use in the crystallization method of the invention is a solvent which is substantially composed of an aliphatic hydrocarbon solvent and/or an aromatic hydrocarbon solvent. Thus, it may be an aliphatic hydrocarbon solvent or an aromatic hydrocarbon solvent, or an aliphatic hydrocarbon solvent and an aromatic hydrocarbon solvent may be used in combination. The term "substantially" as used herein means that a solvent other than the aliphatic or aromatic hydrocarbon solvent may be coexist within the range not causing adverse effects. Specifically, though depending on the kind of other coexisting solvent, the sum of the aliphatic hydrocarbon solvent and aromatic hydrocarbon solvent is not less than 80 volume %, preferably not less than 90 volume %, more preferably not less than 95 volume % relative to the total solvent amount. From crystallizing concentration points of view, it is preferable to use an aromatic hydrocarbon solvent or an aliphatic hydrocarbon solvent and an aromatic hydrocarbon solvent in combination. Generally, however, with emphasis on crystallization yield and workability such as fluidity, it is preferable to use an aliphatic hydrocarbon solvent or an aliphatic hydrocarbon solvent and an aromatic hydrocarbon solvent in combination.

In the crystallization method of the invention, when the proportion of an aromatic hydrocarbon solvent is large, the crystallization yield tends to be lowered. Therefore, the ratio of the two solvents, as described in the volume ratio of the aromatic hydrocarbon solvent to the aliphatic hydrocarbon solvent at completion of crystallization, is generally not more than 1, preferably not more than 2/3, more preferably not more than 1/2, although the ratio may be appropriately varied considering the objective quality. Generally, the crystallization can be carried out advantageously with the above-mentioned volume ratio of generally not more than 1/3, preferably within the range of 1/20 to 1/3.

The aliphatic hydrocarbon solvent for use in the crystallization method of the invention is not particularly restricted but includes, for example, chain or cyclic aliphatic hydrocarbons containing 5 to 8 carbon atoms. Specifically, for example, hexane, heptane, octane, cyclohexane, methylcyclohexane, ethylcyclohexane, etc. can be mentioned. Preferred are hexane and methylcyclohexane. When an aromatic hydrocarbon solvent is not concomitantly used, it is preferable to use an aliphatic hydrocarbon solvent having a comparatively high boiling point, such as methylcyclohexane, for the purpose of dissolving the solute under warming to a high concentration.

The aromatic hydrocarbon solvent for use in the crystallization method of the invention is not particularly restricted but includes, for example, monocyclic aromatic hydrocarbons containing 6 to 10 carbon atoms. Specifically, for example, benzene, toluene, o-xylene, m-xylene, p-xylene, ethylbenzene, cumene, and t-butylbenzene can be mentioned. Preferred is toluene. When an aliphatic hydrocarbon solvent is not concomitantly used, it is preferable to use a more lipophilic aromatic hydrocarbon solvent from crystallization yield points of view.

The crystallization method of the present invention is not particularly restricted but includes, for example, crystallization method by cooling, crystallization method by concentration, crystallization method by solvent exchange (inclusive of the crystallization method in which a solution of a solvent other than said hydrocarbon solvent is replaced partially or totally with a solution of said hydrocarbon solvent, or the crystallization method in which a solution of an aromatic hydrocarbon solvent is replaced partially or totally with a solution of an aliphatic hydrocarbon solvent), crystallization method by adding a solution of an aromatic hydrocarbon solvent containing 2-acetylthio-3-phenylpropionic acid into an aliphatic hydrocarbon solvent, and crystallization method by adding an aliphatic hydrocarbon solvent to a solution of an aromatic hydrocarbon solvent containing 2-acetylthio-3-phenylpropionic acid. From the standpoint of the purity of the resulting 2-acetylthio-3-phenylpropionic acid crystals, the crystallization method by cooling is optimal and the crystallization can advantageously be carried out by using the crystallization method by cooling in combination with another crystallization method as well.

In carrying out the crystallization, seed crystals may be added where necessary.

The crystallization concentration (the weight of 2-acetylthio-3-phenylpropionic acid relative to the volume of the solvent) at completion of crystallization is not particularly restricted but, for example, maybe 1 to 50 w/v%, however, since the lower the concentration tends to be preferred from viewpoints of the inhibition of oil formation and the purity of resulting crystals, crystallization can be advantageously carried out at not over 30 w/v %, more preferably not over 20 w/v %. The lower limit of the crystallization concentration may be not less than 1 w/v %, for example, but from productivity points of view, it is generally not less than 2 w/v %, preferably not less than 3 w/v %, particularly not less than 5 w/v %. The crystallization concentration may be appropriately varied in consideration of the objective quality.

The crystallization temperature is generally not higher than 50°C and there is no particular limitation but, generally, as a simple method, the amount of the crystals can be increased by carrying out crystallization at about 20 to 30°C, for example, and finally cooling to a temperature not higher than 10°C or not higher than 0°C.

The intensity of agitation during crystallization is not particularly restricted but from mild agitation to intense agitation may be employed. Generally, however, the crystallization can be carried out with advantage under moderate agitation to intense agitation. More particularly, as the stirring intensity per unit volume, the stirring can be advantageously carried out for example, not less than 0.1 kW/m$^3$, generally not less than 0.2 kW/m$^3$.

In the crystallization method of the invention, various impurities inclusive of, for example, unreacted 2-halo-3-phenylpropionic acid, byproduct 2-mercapto-3-phenylpropionic acid or its disulfide form, and the enantiomer of 2-acetylthio-3-phenylpropionic acid can be efficiently removed.

After completion of crystallization, the crystals can be isolated by the conventional solid-liquid separation methods such as centrifugation, pressure filtration, suction filtration, etc. and, where necessary, washed with, for example, an aliphatic hydrocarbon solvent and/or an aromatic hydrocarbon solvent (e.g. a solvent identical in composition to the aliphatic hydrocarbon solvent and/or aromatic hydrocarbon solvent in the mother liquor at completion of crystallization). Furthermore, the crystals can be dried under reduced pressure (vacuum) at a temperature not higher than about 40°C, for instance, to give dry crystals.

By the crystallization method of the present invention, 2-acetylthio-3-phenylpropionic acid can be isolated and purified as crystals of the free acid in a good condition.

BEST MODE FOR CARRYING OUT THE INVENTION

The following examples illustrate the present invention in further detail however, they are by no means limitative to the scope of the invention.

The assay of 2-acetylthio-3-phenylpropionic acid and the evaluation of apparent purity and impurity content were performed by the following HPLC analyses.

(HPLC1: assay)

[Column; Nomura Chemical's Dovelosil ODS-HG-3, 150 mm × 4.6 mm I. D.; mobile phase: 0.1 wt/v % aqueous phosphoric acid solution/acetonitrile = 75/25; flow rate: 1.0 ml/min.; detection: UV 210 nm; column temperature: 40°C; retention time: 2-mercapto-3-phenylpropionic acid 16.1 min.; 2-chloro-3-phenylpropionic acid 19.9 min.; 2-acetylthio-3-phenylpropionic acid 22.6 min.]

(HPLC2: apparent purity and impurity content)

[Column; Nomura Chemical's Develosil ODS-HG-3, 150 mm × 4.6 mm I. D.; mobile phase: 0.1 wt/v % aqueous phosphoric acid solution/acetonitrile = 40/60; flow rate: 1.0 ml/min.; detection: UV 210 nm; column temperature: 40°C; retention time: 2-acetylthio-3-phenylpropionic acid 2.8 min.]

The apparent purity, in the above assay system (HPLC2), is expressed by the following formulation (1) :

$$\text{Apparent purity (\%) = (the peak area of}$$

$$\text{2-acetylthio-3-phenylpropionic acid/the sum of peak areas of}$$

$$\text{all detected compounds)} \times 100 \tag{1}$$

The apparent purity (exclusive of the solvent) as described herein below, in the above assay system (HPLC2), is expressed by the following formulation (2):

$$\text{Apparent purity (exclusive of the solvent) (\%) = \{the peak area}$$

$$\text{of 2-acetylthio-3-phenylpropionic acid/((the sum of peak areas}$$

$$\text{of all detected compounds - the peak area of the solvent)\}} \times 100 \tag{2}$$

The evaluation of the optical purity of 2-acetylthio-3-phenylpropionic acid was determined after derivatization to the corresponding methyl ester as follows. Thus, 25 mg (0.12 mmol) of the product was dissolved in the mixed solution composed of 1 ml of methanol and 3.5 ml of toluene, and 166 mg (0.15 mmol) of 10% trimethylsilyldiazomethane/hexane solution was added dropwise and reacted at room temperature for 30 minutes. The solvent was then distilled off under reduced pressure and the concentrate was purified by silica gel column (hexane/ethyl acetate = 4: 1) to give methyl 2-acetylthio-3-phenylpropionate. HPLC analysis of this methyl ester [column: Chiralcel OD-H (product of Daicel), eluent: hexane/isopropanol=100:1, flow rate: 1.0ml/min., temperature: 40°C, detection wavelength: 210 nm, retention time: R-form 37 min., S-form 38 min.]

Example 1

In 162 ml of dimethylformamide was dissolved 80.9 g (0.4383 mole) of (R)-2-chloro-3-phenylpropionic acid. Then, 66.53 g (0.5829 mole) of potassium thioacetate was added slowly at 10 to 20°C with stirring under nitrogen atmosphere and the mixture was further stirred at room temperature for 48 hours. After 270 ml of 6% sodium thiosulfate aqueous solution was added at 20 to 25°C, 630 ml of toluene was added. Then, 19.2 g of concentrated hydrochloric acid was added gradually to lower the pH from 5.5 to 2.0. The organic phase was separated to give an organic phase 1. The remaining aqueous phase was extracted with 630 ml of toluene to give an organic phase 2. Substantially all the (S)-2-acetylthio-3-phenylpropionic acid produced was present in the organic phase 1. The organic phase in which organic phases 1 and 2 are combined ((S)-2-acetylthio-3-phenylpropionic acid content: 82.79 g (84.2 mole %)) was washed serially with 270 ml of 6 wt. % sodium thiosulfate aqueous solution, 800 ml × 3 times of 20 wt. % sodium chloride aqueous solution, and 800 ml × 2 times of pure water. From the washed organic phase ((S)-2-acetylthio-3-phenylpropionic acid content: 81.93 g (83.3 mole %)), the equivalent of 14.30 g of (S)-2-acetylthio-3-phenylpropionic acid was taken and concentrated under reduced pressure to give 24.81 g of a concentrate (apparent purity (exclusive of solvent) : 90%). To this concentrate was added 23.5 ml of toluene, and after mixing, 107 ml of hexane was added dropwise at 20°C. Crystals were precipitated in the course of dropwise addition. After completion of the dropwise addition of hexane, a small amount of the precipitated crystals was taken and washed with a small quantity of hexane/toluene (75/25, in volume ratio). The apparent purity was 97%. The slurry was heated to 40°C so as to dissolve the precipitated crystals. The resulting solution was allowed to cool gradually under stirring, whereupon crystals were precipitated already at an internal temperature of about 30°C. The system was further cooled to about 15°C over about 3 hours. The crystals thus obtained were subjected to suction filtration and washed with 50 ml of hexane/toluene (75/25, in volume ratio). The thus-obtained wet crystals contained 11.44 g of (S)-2-acetylthio-3-phenylpropionic acid (apparent purity (exclusive of solvent): 98%).

From these wet crystals, the equivalent of 4.96 g of (S)-2-acetylthio-3-phenylpropionic acid was taken and dissolved by adding 12.5 ml of toluene under warming and 37.5 ml of hexane was added dropwise at 20°C. After completion of the dropwise addition of hexane, the slurry was heated to 40°C to dissolve the precipitated crystals. This solution was allowed to cool overnight under stirring to about 10°C. The obtained crystals were subjected to suction filtration and washed with 20 ml of hexane/toluene (75/25, in volume ratio) . The wet crystals thus obtained contained 4.02 g of (S)-2-acetylthio-3-phenylpropionic acid (apparent purity (exclusive of solvent): not less than 99%).

Example 2

Using ethyl acetate as the extraction solvent, the procedure of Example 1 was otherwise followed to give a concentrate containing (S)-2-acetylthio-3-phenylpropionic acid. The solvent was further distilled off under reduced pressure to give a concentrate ((S)-2-acetylthio-3-phenylpropionicacidconcentration: ca 75 wt. %, apparent purity: 91%). A 6 g portion of this concentrate was mixed with 5 ml of toluene and seed crystals were added under ice-cooling for crystallization. Then, 45 ml of hexane was added gradually at an internal temperature of 5 to 25°C. A portion of the precipitated crystals was taken and washed with hexane. The apparent purity (exclusive of solvent) of the crystals thus obtained was 96%.

Example 3

The concentrate used in Example 2 (6 g) was mixed with 15 ml of toluene and 100 ml of hexane and the mixture was warmed on a water bath (60°C) to be dissolved completely, which was, then, cooled gradually to 20°C. A portion of the precipitated crystals was taken and washed with hexane. The apparent purity (exclusive of solvent) of the crystals thus obtained was 99%.

Example 4

The concentrate used in Example 2 (6 g) was mixed with 100 ml of methylcyclohexane and the mixture was warmed on a water bath (about 50°C) to be dissolved completely, which was, then, cooled gradually to 20°C. A portion of the precipitated crystals was taken and washed with hexane. The apparent purity (exclusive of solvent) of the crystals thus obtained was 98%.

Example 5

The concentrate containing (S)-2-acetylthio-3-phenyl-propionic acid (equivalent to 500 mg of (S)-2-acetylthio-3-phenylpropionic acid) as obtained by the same procedure as in Example 2 was mixed with 300 mg of toluene and crystallized by adding seed crystals under cooling at -10°C. Then, under ice-cooling, 5 ml of hexane was gradually added, whereupon the slurry was crystallized uneventfully without developing an oil.

Comparative Example 1

The concentrate used in Example 5 (equivalent to 500 mg of (S)-2-acetylthio-3-phenylpropionic acid) was mixed with 300 mg of ethyl acetate, followed by gradual addition of 5 ml of hexane. At the point of time when the solution became white and turbid, 10 mg of seed crystals were added but the solution became oily.

Example 6

The concentrate containing (S)-2-acetylthio-3-phenyl-propionic acid as obtained by the same procedure as in Example 2 was subjected to solvent exchange with toluene to give a 50 wt. % solution of (S)-2-acetylthio-3-phenyl-propionic acid in toluene. This solution (equivalent to 20 g of (S)-2-acetylthio-3-phenylpropionic acid) was ice-cooled and a small amount of seed crystals was added to precipitate (S)-2-acetylthio-3-phenylpropionic acid. The slurry was then warmed to room temperature and under moderate stirring, 200 ml of hexane was added over 10 hours. Suction filtration was carried out to collect wet crystals, which were then thoroughly washed with hexane. The crystallization yield was 90%.

Example 7

In 10 ml of hexane was dissolved crystals of (S)-2-acetylthio-3-phenylpropionic acid at an internal temperature of about 60°C until substantial saturation was obtained. This solution was cooled gradually to room temperature, whereupon crystals were precipitated.

Example 8

To a mixed solution composed of 8 ml of toluene and 32 ml of hexane was added 4.0 g of crystals of (S)-2-acetylthio-3-phenylpropionic acid (apparent purity: 95%), and the mixture was warmed for dissolution at about 50°C. This solution was cooled gradually to room temperature to give a slurry. The crystals thus obtained were subjected to suction filtration and washed with 10 ml of hexane to give wet crystals containing 3.2 g of (S)-2-acetylthio-3-phenyl-propionic acid (apparent purity (exclusive of solvent): not less than 99%).

Comparative Example 2

To a mixed solution composed of 3 ml of methyl t-butyl ether and 37 ml of hexane was added 4.0 g of crystals of (S)-2-acetylthio-3-phenylpropionic acid (apparent purity 95%), and the mixture was warmed to about 50°C, whereupon a solution separated into two phases was obtained. To this solution was added 7 ml of methyl t-butyl ether, and the solution was homogenized at about 50°C. With occasional addition of seed crystals, the above solution was allowed to cool to room temperature but an oil was obtained. This oil did not solidify when being allowed to stand overnight.

Comparative Example 3

To a mixed solution composed of 3 ml of ethyl acetate and 37 ml of hexane was added 4.0 g of crystals of (S)-2-acetylthio-3-phenylpropionic acid (apparentpurity: 95%), and the mixture was warmed to about 50°C, whereupon a solution separated into two phases was obtained. To this solution was added 2 ml of ethyl acetate, and the mixture was homogenized at about 50°C. With occasional addition of seed crystals, the above solution was allowed to cool to room temperature but an oil was obtained. When this oil was allowed to stand overnight, it solidified but adhered extensively to the vessel wall. When this slurry was subjected to suction filtration, it turned into an oil.

Example 9

To 6 ml of t-butylbenzene was added 2.0 g of crystals of (S)-2-acetylthio-3-phenylpropionic acid (apparent purity: 95%), followed by heating to about 50°C for dissolution. The resulting solution was gradually cooled to room temperature to give a slurry. The crystals thus obtained were subjected to suction filtration and washed with 6 ml of t-butylbenzene to give wet crystals containing 0.3 g of (S)-2-acetylthio-3-phenylpropionic acid (apparent purity (exclusive of solvent): not less than 99%).

Example 10

To 100 ml of methylcyclohexane was added 4.0 g of crystals of (S)-2-acetylthio-3-phenylpropionic acid (apparent purity: 95%), followed by heating to about 50°C for dissolution. The resulting solution was gradually cooled to room temperature to give a slurry. The crystals thus obtained were subjected to suction filtration and washed with 10 ml of methylcyclohexane to give wet crystals containing 3.3 g of (S)-2-acetylthio-3-phenylpropionic acid (apparent purity (exclusive of solvent): 98%).

Example 11

In this example, for the purpose of obtaining extremely high quality crystals of (S)-2-acetylthio-3-phenylpropionic acid, particularly for minimizing the hardly removable impurity corresponding to a retention time of 3.9 min. (HPLC2), the reaction mixture was incubated for ripening and, then, subjected to crystallization under the defined conditions.

To 632 ml of dimethylformamide was added 266.2 g (2.331 moles) of potassium thioacetate, and 331.0 g (1.793 moles) of (R)-2-chloro-3-phenylpropionic acid was added over 30 minutes at 15 to 25°C under nitrogen. The mixture was stirred at 20 to 25°C for 3 hours (% residue of (R)-2-chloro-3-phenylpropionic acid: 6.0%; the content of the impurity corresponding to a retention time of 3.9 min.: 3.4%).

The mixture was further stirred at 20 to 30°C for 22 hours (% residue of (R)-2-chloro-3-phenylpropionic acid: less than 0.1%; the content of the impurity corresponding to a retention time of 3.9 min.: 0.7%).

The reaction mixture was further incubated for ripening at 7 to 20°C for 21 hours (% residue of (R)-2-chloro-3-phenyl-propionic acid: less than 0.1%; the content of the impurity corresponding to a retention time of 3.9 min.: 0.3%).

This reaction solution was then treated as the same manner as in Example 1 to give 475.2 g of a concentrate containing 341.8 g of (S)-2-acetylthio-3-phenylpropionic acid (apparent purity (exclusive of solvent): 85%, the content of the impurity corresponding to a retention time of 3.9 min.: 0.3%, optical purity: 98.6% e.e.).

This concentrate was mixed with 3418 ml of toluene/hexane (25/75, in volume ratio) and dissolved by warming at 30°C, followed by addition of seed crystals. At 27°C, the solution began to develop white turbidity and crystallize. The crystallization was further allowed to proceed at 20°C for 2 hours. The resulting crystals were subjected to suction filtration and washed with 690 ml of toluene/hexane (25/75, in volume ratio). The wet crystals thus obtained were dried under reduced (vacuum) pressure (full vacuum, 20 to 40°C, overnight) to give 219.8 g of (S)-2-acetylthio-3-phenylpropionic acid (apparent purity: not less than 99%, the content of the impurity corresponding to a retention time of 3.9 min.: not more than 0.1%, optical purity: not less than 99.8% e.e.).

Example 12

To 20 ml of dimethyl formamide was added 8.04 g (70.4 mmole) of potassium thioacetate, 10.0 g (50.4 mmole) of (R)-2-chloro-3-phenylpropionic acid was added over 30 minutes at 15 to 25°C under nitrogen atmosphere, and the mixture was further stirred for 4 hours. (% residue of (R)-2-chloro-3-phenylpropionic acid: 4.0%, (S)-2-mercapto-3-phenylpropionic acid content: 0.5%).

This reaction mixture was treated as the same manner as in Example 1 to give 11.4 g of a concentrate containing 8.2 g of (S)-2-acetylthio-3-phenylpropionic acid ((R)-2-chloro-3-phenylpropionic acid content: 4.0%; (S)-2-mercapto-3-phenylpropionic acid content: 0.5%). This concentrate was crystallized under the same conditions as in Example 11. As a result, 5.3 g of (S)-2-acetylthio-3-phenylpropionic acid was obtained ((R)-2-chloro-3-phenylpropionic acid content: not more than 0.1%; (S)-2-mercapto-3-phenylpropionic acid: not detected).

INDUSTRIAL APPLICABILITY

In accordance with the present invention constituted as above, 2-acetylthio-3-phenylpropionic acid which is important for the production of pharmaceuticals and the like can be recovered and purified as crystals of the free acid.

**Claims**

1. A crystallization method of 2-acetylthio-3-phenylpropionic acid
which comprises crystallizing 2-acetylthio-3-phenylpropionic acid by using a solvent substantially composed of an aliphatic hydrocarbon solvent and/or an aromatic hydrocarbon solvent as a crystallization solvent.

2. The crystallization method according to Claim 1,
wherein a volume ratio of the aromatic hydrocarbon solvent to the aliphatic hydrocarbon solvent at completion of crystallization is not more than 1.

3. The crystallization method according to Claim 1 or 2,
wherein the crystallization is carried out by using the aliphatic hydrocarbon solvent and the aromatic hydrocarbon solvent in combination.

4. The crystallization method according to Claim 1, 2 or 3,

wherein the crystallization is carried out by adding the aliphatic hydrocarbon solvent to a solution of the aromatic hydrocarbon solvent containing 2-acetylthio-3-phenylpropionic acid.

5. The crystallization method according to Claim 4,
wherein the solution of the aromatic hydrocarbon solvent containing 2-acetylthio-3-phenylpropionic acid is a solution obtainable by reacting 2-halo-3-phenylpropionic acid with a salt of thioacetic acid to synthesize 2-acetylthio-3-phenyl-propionic acid and extracting 2-acetylthio-3-phenylpropionic acid with the aromatic hydrocarbon solvent under acidic conditions, or a concentrate thereof, or a solution obtainable by extracting 2-acetylthio-3-phenylpropionic acid with an organic solvent other than the aromatic hydrocarbon solvent under acidic conditions and subjecting the resulting solution to solvent exchange to ultimately give a solution in the aromatic hydrocarbon solvent, or a concentrate thereof.

6. The crystallization method according to Claim 1 or 2,
wherein the aliphatic hydrocarbon solvent is used while the aromatic hydrocarbon solvent is not used in combination therewith.

7. The crystallization method according to Claim 1, 2, 3, 4 or 5,
wherein the aromatic hydrocarbon solvent is a monocyclic aromatic hydrocarbon containing 6 to 10 carbon atoms.

8. The crystallization method according to Claim 7,
wherein the monocyclic aromatic hydrocarbon containing 6 to 10 carbon atoms is toluene.

9. The crystallization method according to Claim 1, 2, 3, 4, 5, 6, 7 or 8,
wherein the aliphatic hydrocarbon solvent is a chain or cyclic aliphatic hydrocarbon containing 5 to 8 carbon atoms.

10. The crystallization method according to Claim 9,
wherein the chain or cyclic aliphatic hydrocarbon containing 5 to 8 carbon atoms is hexane or methylcyclohexane.

11. The crystallization method according to Claim 1, 2, 3, 4, 5, 6, 7, 8, 9 or 10,
wherein the crystallization method is a crystallization method by cooling.

12. The crystallization method according to Claim 1, 2, 3, 4, 5, 6, 7, 8, 9, 10 or 11,
wherein the crystallization is carried out at not higher than 50°C.

13. The crystallization method according to Claim 1, 2, 3, 4, 5, 6, 7, 8, 9, 10, 11 or 12,
wherein a seed crystal is added at the crystallization.

14. The crystallization method according to Claim 1, 2, 3, 4, 5, 6, 7, 8, 9, 10, 11, 12 or 13,
wherein 2-acetylthio-3-phenylpropionic acid as obtained by ripening a reaction solution to reduce an amount of an impurity difficult to be removed by the crystallization at a reaction of 2-halo-3-phenylpropionic acid with a salt of thioacetic acid is used in the crystallization.

<table>
<tr><td colspan="2" align="center">**INTERNATIONAL SEARCH REPORT**</td><td>International application No.<br>PCT/JP01/03383</td></tr>
</table>

| A. CLASSIFICATION OF SUBJECT MATTER |
|---|
| Int.Cl$^7$   C07C327/32 |
| According to International Patent Classification (IPC) or to both national classification and IPC |

| B. FIELDS SEARCHED |
|---|
| Minimum documentation searched (classification system followed by classification symbols)<br>Int.Cl$^7$   C07C327/00 |
| Documentation searched other than minimum documentation to the extent that such documents are included in the fields searched |
| Electronic data base consulted during the international search (name of data base and, where practicable, search terms used)<br>REGISTRY (STN), CA (STN) |

**C. DOCUMENTS CONSIDERED TO BE RELEVANT**

| Category* | Citation of document, with indication, where appropriate, of the relevant passages | Relevant to claim No. |
|---|---|---|
| P,X | JP 2000-309557 A (Ajinomoto Co., Inc.), 07 November, 2000 (07.11.00), pages 2 to 3   (Family: none) | 1-14 |
| Y | EP 747392 A1 (Bristol-Myers Squibb Co.), 11 December, 1996 (11.12.96), & JP, 8-337527, A | 1-14 |
| Y | JP 61-92585 A (Mitsubishi Rayon Co., Ltd.), 10 May, 1986 (10.05.86), pages 1 to 2   (Family: none) | 1-14 |

| ☐ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>26 June, 2001 (26.06.01) | Date of mailing of the international search report<br>17 July, 2001 (17.07.01) |
|---|---|
| Name and mailing address of the ISA/<br>Japanese Patent Office | Authorized officer |
| Facsimile No. | Telephone No. |

Form PCT/ISA/210 (second sheet) (July 1992)